# EUROPEAN PATENT APPLICATION

(11) **EP 2 238 994 A1**
(43) Date of publication of application: **13.10.2010**
(21) Application number: 09157456.6
(22) Date of filing: 07.04.2009
(51) Int. Cl.: A61M 1/06

(54) **Breast pump insert**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

A breast pump insert to receive the breast of a user of a breast pump is disclosed. The insert is formed from inner and outer materials, wherein the outer material exhibits greater hardness than the inner material.

## Description

### FIELD OF THE INVENTION

The present invention relates to an insert for a breast pump.

### BACKGROUND OF THE INVENTION

Breast pumps are well known devices for extracting milk from a breast of a user. A breast pump may be used if the baby or infant is not itself able to extract milk from the breast, or if the mother is separated from the baby or infant, for example, if away from the baby at work. Breast pumps typically comprise a rigid funnel connected to a vacuum pump having a container for collecting the milk. A soft elastomer insert is generally disposed in the rigid funnel which adapts to the contour of the breast so as to provide comfort and a vacuum seal necessary for operating the pump. It is also known from US20040029486 to provide an insert having an elastic wall which forms a chamber together with an outer frame such that the chamber can hold deformable medium including gas, liquids or solids to further improve the comfort during pumping.

Moreover, to provide comfort and optimal pumping it is also important that the vacuum seal is maintained. To obtain the most efficient vacuum it is beneficial if the breast easily slides into the insert positioned within the funnel. However, conventional elastomer inserts have a high friction coefficient due to their softness causing the skin of the breast to rub against the insert which may result in friction trauma. This condition may be exacerbated when using breast pumps exerting a peristaltic movement on the breast and/or nipple to stimulate expression of milk, as the peristaltic movement of the insert creates a greater dynamic contact between the skin and the insert exposing the skin to further friction compared to breast pumps merely exerting a negative pressure on the breast tissue.

### SUMMARY OF THE INVENTION

The present invention seeks to overcome or substantially alleviate the aforementioned problems to provide an insert for use in breast pumps.

Accordingly, the present invention provides a breast pump insert to receive the breast of a user of a breast pump, said insert being formed from inner and outer materials, wherein the outer material exhibits greater hardness than the inner material.

Preferably, the inner material is made from a resilient and/or compliant material.

Conveniently, the outer material is flexible such that movement or deformation of the inner material is transferred through the outer material.

In one embodiment, the outer material has a thickness less than 0.2mm.

In an alternative embodiment, the outer material has a thickness up to 0.5mm.

The outer material may entirely enclose the inner material.

Conveniently, the outer material extends across a part of a surface of the inner material.

Preferably, the entire outer material is attached to a surface of the inner material.

In one embodiment, a portion of the outer material is attached to the inner material.

In an alternative embodiment, the entire outer material is separable from the inner material.

The inner material may comprise a solid.

Conveniently, the inner material comprises a non-solid such as a gel, fluid or gas which is retained by the outer material.

### BRIEF DESCRIPTION OF THE DRAWINGS

Preferred embodiments will now be described, by way of example only, with reference to the accompanying drawings, in which:
Figure 1 shows a cross section of an insert disposed in a funnel according to one embodiment of the present invention; and
Figure 2 shows a cross-section of an insert disposed in a funnel according to another embodiment of the present invention.

### DETAILED DESCRIPTION OF THE EMBODIMENTS

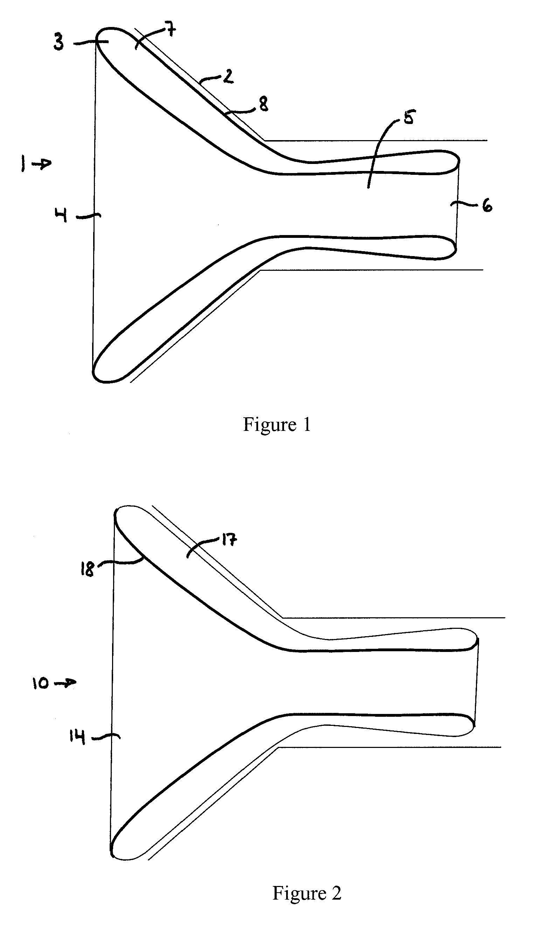

Referring now to the drawings, Figure 1 illustrates an embodiment of an insert 1 which is disposed in a funnel 2 of a breast pump for extracting milk. The insert 1 comprises a frustoconical shaped wall 3 having a large opening 4 opposing a narrow passage 5 leading to a small opening 6. Although not illustrated in Figure 1, the insert 1 fits neatly into the funnel 2 so as to form an airtight seal as the breast is received in the insert 1.

The wall 3 of the insert 1 comprises an inner soft material 7 which forms the bulk of the insert 1 and an outer hard material 8 enclosing said inner portion 7. The inner soft portion 7 is formed of a compressible and resilient material such that it easily deforms and adapts to the contour of the breast. Preferably, the inner soft material 7 comprises a silicone gel or silicone rubber having a hardness up to 40 Shore A, however it is not limited to this material as it can be made of any material having resilient, deformable properties that will provide comfort to the user and achieve a vacuum by adapting to the shape of the breast. Preferably, the inner soft material 7 has a thickness of 1 to 5mm; however particular regions can be made thicker if so needed. The configuration of the insert shown in Figure 1 enables the inner soft material 7 to be made of a non-solid as well as a solid, since any fluid, gel or gas can be contained within the enclosing outer hard material 8.

The outer hard material 8 can be made of any plastic material having a low friction coefficient, for example, polyurethane, polypropylene, polyethylene, Teflon and Nylon or an elastomer having a hardness greater than 60 shore A. The low friction coefficient of the outer hard material 8 gives the insert 1 its smooth characteristics and enables the breast to easily slide into the insert 1 without the discomfort caused by the greater friction of elastomers used in conventional inserts. The thickness of the outer hard material 8 should allow it to be flexible so that it does not mask the elastic properties of the underlying soft inner material 7. Therefore, appropriate thickness of the outer hard material 8 should be thinner than 0.2mm or, when the soft inner material 7 is easily deformed, it might be feasible for the outer material 8 to have a thickness up to 0.5mm.

It will be appreciated that the combination of the different material properties and the configuration of the insert provide significant advantages over conventional inserts in that the resilient property as provided by the soft inner material 7 is maintained owing to the specific thickness of the outer material and so enables the insert 1 to adapt to the contours of the breast. The low friction of the outer material facilitates in the breast easily being received in the insert so as to achieve a smooth resilient insert further assisting in obtaining vacuum when the pump is operated.

In an alternative embodiment of the present invention, an insert comprises an outer hard material which only extends across part of a surface of an inner soft material, the two materials being formed from two solids. For example, Figure 2 illustrates an insert 10 having an outer hard material 18 which covers the surface of a soft inner material 17 which is in contact with the skin of the breast during operation of the pump. This configuration reduces the friction between the skin and the insert whilst a greater degree of friction is maintained between the insert and the funnel so that the insert is securely positioned in the funnel during use. Moreover, it should be realized that in un-illustrated embodiments the outer hard material extends across the inner soft material to a greater or smaller extent than what is illustrated in Figure 2.

It is envisaged that when the inner soft material and the outer hard material as previously described are made from solids, they may be completely joined to one another or partially attached in regions via chemical or mechanical coupling. For example, the outer hard material 18 extending across a part of the surface of the inner soft material 17 as shown in Figure 2, may be attached to one another at the large opening 14. An alternative configuration to the embodiment illustrated in Figure 2 includes the hard and soft materials being completely separable enabling either to be replaced if so needed or to provide an outer hard layer to an already existing insert having a high friction coefficient.

In the aforementioned embodiments, the components forming the outer hard and inner soft materials can be premade prior to forming the insert. Alternatively, the outer layer can initially be in a liquid form as it is applied to the inner soft material and thereafter harden. This enables the outer material to coat regions as well as the entire inner material.

It should be realized that the smooth feeling provided by the outer material can be further improved by decreasing its surface roughness so as to further reduce the friction. Alternatively, the surface of the outer material can be roughened, giving it silk-like properties, thereby reducing the friction between the skin and the insert.

The invention has been described with reference to preferred embodiments only. Modifications and alterations to the embodiments falling within the scope of the appended claims are included within the scope of protection.

## Claims

1. A breast pump insert to receive the breast of a user of a breast pump, said insert being formed from inner and outer materials, wherein the outer material exhibits greater hardness than the inner material.

2. A breast pump insert according to claim 1, wherein the inner material is made from a resilient and/or compliant material.

3. A breast pump insert according to claims 1 and 2, wherein the outer material is flexible such that movement or deformation of the inner material is transferred through the outer material.

4. A breast pump insert according to claims 1 to 3, wherein the outer material has a thickness less than 0.2mm.

5. A breast pump insert according to claims 1 to 3, wherein the outer material has a thickness up to 0.5mm.

6. A breast pump insert according to any preceding claim, wherein the outer material entirely encloses the inner material.

7. A breast pump insert according to claims 1 to 5, wherein the outer material extends across a part of a surface of the inner material.

8. A breast pump insert according to claim 6 and 7, wherein the entire outer material is attached to a surface of the inner material.

9. A breast pump insert according to claim 6 and 7, wherein a portion of the outer material is attached to the inner material.

10. A breast pump insert according to claim 7, wherein the entire outer material is separable from the inner material.

11. A breast pump insert according to any preceding claim, wherein the inner material comprises a solid.

12. A breast pump insert according to claim 1 to 6, wherein the inner material comprises a non-solid such as a gel, fluid or gas which is retained by the outer material.
